# FASCICULE DE BREVET EUROPEEN

(11) **EP 0 764 312 B1**
(45) Date de publication et mention de la délivrance du brevet: **15.04.1998**
(21) Numéro de dépôt: 95922587.1
(22) Date de dépôt: 09.06.1995
(51) Int. Cl.: G06M 1/16, G06M 1/04

(54) **COMPTEUR DE DOSES POUR INHALATEURS**
DOSENZÄHLER FÜR INHALATOREN
DOSE COUNTING DEVICE FOR INHALATORS

(30) Priorité: 10.06.1994 FR 9407114
(43) Date de publication de la demande: 26.03.1997
(73) Titulaire: VALOIS S.A., 27110 Le Neubourg (FR)
(72) Inventeur: BRUNA, Pascal, F-76000 Rouen (FR)
(74) Mandataire: CAPRI SARL
(86) Numéro de dépôt international: FR9500756
(87) Numéro de publication internationale: WO9534874

(56) Documents cités:
- EP-A- 0 254 391
- DE-A- 48 610
- FR-A- 819 885
- FR-A- 1 514 296
- FR-A- 2 103 434
- FR-A- 2 341 166

## Description

La présente invention concerne un dispositif de comptage des doses émises par un distributeur de produit, et plus particulièrement par un inhalateur, tel qu'on en utilise par exemple dans le domaine de la pharmacie.

Dans les applications médicales en particulier, il est souvent nécessaire de pulvériser un certain nombre de doses du produit contenu dans le distributeur, par exemple quotidiennement. Pour éviter les erreurs de manipulations et/ou de dosage, il est souhaitable de prévoir des moyens permettant d'afficher le nombre de doses pulvérisées, ou restant à pulvériser si on a affiché préalablement le nombre maximal de doses à pulvériser (compteur ou décompteur).

On a déjà proposé, dans le brevet EP-0 269 496 un compteur de l'actionnement du poussoir comportant un couronne de comptage montée rotative de manière coaxiale au poussoir et munie d'une denture périphérique orientée axialement. Une lame souple solidaire du poussoir entraîne ladite couronne en rotation lors de l'actionnement dudit poussoir. Ce dispositif est simple et peu coûteux mais, du fait qu'il ne comporte qu'une couronne, limite le nombre de doses comptées.

D'autre part, ce dispositif transformant le mouvement axial du poussoir en un mouvement rotatif de la couronne, il n'est pas adapté à fonctionner indépendamment du poussoir d'actionnement.

Le document DE-48610 décrit un dispositif de comptage comprenant plusieurs couronnes montées rotatives autour d'un axe de rotation fixe, la première couronne comportant une languette d'entraînement de la seconde couronne, mobile entre une position de repos et une position d'entraînement, la languette étant forcée dans sa position d'entraînement par un moyen de came.

La présente invention a pour but de fournir un dispositif de comptage de doses capable de compter un nombre quelconque de doses (typiquement de quelques dizaines à quelques centaines de doses) et pouvant être actionné par un organe exerçant un mouvement translatif.

La présente invention a aussi pour but de fournir un dispositif de comptage de doses capable de compter un nombre quelconque de doses (typiquement de quelques dizaines à quelques centaines de doses) et pouvant être actionné par un organe exerçant un mouvement rotatif.

La présente invention a encore pour but de fournir un dispositif de comptage de doses destiné à compter un nombre prédéterminé de doses, et adapté à empêcher l'actionnement du distributeur de produit après que ledit nombre prédéterminé de doses air été compté.
La présente invention a donc pour objet un dispositif de comptage des doses de produit émises par un distributeur de produits fluides ou pulvérulents, comprenant une première couronne de comptage et une seconde couronne de comptage, les deux couronnes de comptage étant montées rotatives autour d'un axe de rotation fixe, ladite première couronne de comptage comportant une denture, disposée circonférentiellement par rapport audit axe de rotation fixe, coopérant avec un organe d'entraînement destiné à faire tourner ladite première couronne de comptage autour dudit axe de rotation fixe à chaque utilisation du distributeur, ladite première couronne de comptage comportant en outre une languette d'entraînement mobile entre une position de repos, où elle ne coopère pas avec ladite seconde couronne de comptage, et une position d'entraînement, où elle coopère avec ladite seconde couronne de comptage pour la faire tourner autour dudit axe de rotation fixe, ladite languette d'entraînement étant forcée dans sa position d'entraînement par un moyen de came, caractérisé en ce que ladite seconde couronne de comptage coopère, après l'émission d'un nombre prédéterminé de doses, avec un moyen de butée fixe qui empêche toute rotation ultérieure de ladite seconde couronne de comptage.

Plus particulièrement, ladite seconde couronne de comptage comporte une série de dents disposée circonférentiellement par rapport audit axe de rotation fixe et ladite languette d'entraînement souple de ladite première couronne de comptage comporte à une extrémité une tête, ladite tête venant s'enclencher dans ladite série de dents de ladite seconde couronne de comptage lorsque ladite languette d'entraînement est dans sa position d'entraînement.

De préférence, ladite première couronne de comptage, agissant comme compteur d'unité, comporte une denture périphérique continue contenant dix dents, lesdites dix dents étant réparties régulièrement autour dudit axe fixe, ladite languette d'entraînement coopérant avec ledit moyen de came pour entraîner en rotation ladite seconde couronne de comptage, agissant comme compteur de dizaines, chaque fois que ladite première couronne de comptage effectue un tour complet autour dudit axe de rotation fixe.

Avantageusement, on prévoit un premier dispositif de blocage agissant sur la première couronne de comptage pour l'empêcher de tourner en sens inverse du sens de rotation imposé par ledit élément d'entraînement, et un second dispositif de blocage agissant sur ladite seconde couronne de comptage pour l'empêcher de tourner en sens inverse du sens de rotation imposé par ladite languette d'entraînement de ladite première couronne de comptage.

Plus particulièrement, ledit second dispositif de blocage comprend une patte souple solidaire de ladite seconde couronne de comptage, et munie à une de ses extrémités d'un ergot, ledit ergot coopérant avec un profil cannelé fixe par rapport à l'axe de rotation pour empêcher ladite second couronne de comptage de tourner dans un sens quelconque lorsque ladite languette d'entraînement de ladite première couronne de comptage est dans sa position de repos. Toute rotation de la seconde couronne de comptage en raison d'éventuels frottements est ainsi évitée.

Avantageusement, ledit profil cannelé fixe qui coopère avec ledit ergot de ladite patte souple dudit second dispositif de blocage comporte un moyen de butée fixe bloquant ledit ergot de ladite patte souple, empêchant ainsi la rotation de ladite seconde couronne de comptage, le nombre maximal de doses émises par le distributeur étant ainsi déterminé par le nombre de cannelures du profil cannelé situées avant ledit moyen de butée.

Ainsi, une seconde couronne de comptage comportant cinq dents limite le nombre de doses émises à quarante-neuf, alors qu'avec vingt dents, le nombre maximal de doses émises est de cent-quatre-vingt dix-neuf.

Selon un premier mode de réalisation de l'invention, une tige est fixement montée sur ledit axe de rotation fixe et les première et seconde couronnes de comptages, sensiblement annulaires, sont montées rotatives sur ladite tige fixe, ladite première couronne de comptage comportant une denture périphérique qui s'étend circonférentiellement par rapport audit axe fixe et dont les dents sont tournées vers l'extérieur, ladite denture coopérant avec un organe d'entraînement solidaire d'un poussoir d'actionnement du distributeur et exerçant un mouvement translatif, ledit élément d'entraînement coopérant à chaque actionnement du poussoir avec une dent de ladite denture pour faire tourner ladite première couronne de comptage autour dudit axe de rotation fixe.

De préférence, on prévoit un premier dispositif de blocage comportant une lame souple fixe qui coopère avec la denture de ladite première couronne de comptage pour empêcher celle-ci de tourner en sens inverse du sens de rotation imposé par ledit organe d'entraînement.

Selon le premier mode de réalisation, ladite première couronne de comptage comporte une languette d'entraînement qui s'étend circonférentiellement par rapport audit axe de rotation fixe et comporte à une extrémité une tête, mobile radialement entre une position de repos, où ladite tête s'étend radialement vers l'extérieur au-delà de la surface annulaire extérieure de la première couronne de comptage, et une position d'entraînement, où ladite tête coopère avec ladite seconde couronne de comptage, ledit moyen de came étant fixe par rapport audit axe de rotation et disposé sans frottement sensiblement contre ladite surface annulaire extérieure de ladite première couronne de comptage au niveau de ladite languette d'entraînement, pour forcer la tête de ladite languette d'entraînement dans sa position d'entraînement chaque fois que ladite première couronne de comptage effectue un tour complet autour dudit axe de rotation fixe.

D'autre part, ladite seconde couronne de comptage comporte de préférence une série de dents s'étendant circonférentiellement par rapport audit axe de rotation fixe et tournées vers l'extérieur, ladite série de dents étant disposée radialement à l'intérieur de ladite languette d'entraînement de ladite première couronne de comptage, de sorte que dans sa position d'entraînement, ladite tête de la languette s'enclenche dans une dent de ladite série de dents pour entraîner ladite seconde couronne de comptage en rotation autour dudit axe de rotation fixe. Si ladite série de dents de la seconde couronne de comptage ne comporte que peu de dents (par exemple, cinq), elle ne s'étendra bien entendu que sur une partie de la circonférnce de ladite seconde couronne de comptage.

Avantageusement, le côté extérieur de ladite tête de la languette d'entraînement a un profil complémentaire au profil dudit moyen de came fixe, et le côté intérieur de ladite tête a un profil complémentaire au profil desdites dents de ladite série de dents de la seconde couronne de comptage.

Selon un second mode de réalisation de l'invention, ladite première couronne de comptage comporte une première denture qui s'étend circonférentiellement par rapport audit axe de rotation fixe et dont les dents, tournées vers l'extérieur, sont réparties régulièrement autour dudit axe fixe, pour coopérer avec un organe d'entraînement exerçant un mouvement rotatif, ledit organe d'entraînement, étant actionné par l'utilisateur au moyen d'un bouton d'actionnement mobile en rotation autour de l'axe de rotation entre une première et une seconde positions extrêmes.

De préférence, ledit organe d'entraînement est annulaire, et monté rotatif autour dudit axe de rotation fixe et comporte un bras souple annulaire, mobile radialement entre une position de repos, où une partie saillante dudit bras s'étend radialement vers l'extérieur au-delà de la surface extérieure annulaire dudit bras, et une position d'entraînement, où ladite partie saillante du bras coopère avec une dent de ladite première denture de ladite première couronne de comptage pour l'entraîner en rotation, ledit bras souple étant forcé dans sa position d'entraînement par le bouton d'actionnement.

Avantageusement, ledit bouton d'actionnement est annulaire et monté rotatif autour dudit axe de rotation fixe de manière à entourer ledit organe d'entraînement, ledit bouton d'actionnement comportant des moyens pour faire tourner ledit organe d'entraînement autour dudit axe de rotation fixe et des moyens pour forcer ledit bras dans sa position d'entraînement.

En particulier, lesdits moyens pour forcer ledit bras dans sa position d'entraînement comportent un renflement disposé sur la face annulaire interne dudit bouton d'actionnement, et lesdits moyens pour faire tourner l'organe d'entraînement comportent deux bossages qui coopèrent avec ledit organe d'entraînement, les deux bossages étant disposés à même hauteur sur la face annulaire interne dudit bouton d'actionnement, le premier bossage étant adapté à faire tourner l'organe d'entraînement dans un sens pour amener la partie saillante de son bras en face d'une dent de ladite première denture, et le deuxième bossage étant adapté à entraîner l'organe d'entraînement dans l'autre sens pour faire tourner ladite première couronne de comptage, lorsque le bras souple est dans sa position d'entraînement.

Plus précisément, ladite première denture de ladite première couronne de comptage comporte dix dents, la distance angulaire entre les deux positions extrêmes du bouton d'actionnement est d'environ 180°, et lesdits bossages sont disposés à un espacement angulaire d'environ 144° l'un de l'autre, ledit bouton d'actionnement étant d'abord tourné de 180° dans un sens vers sa seconde position extrême pour amener ladite partie saillante du bras de l'organe d'entraînement en face de la dent suivante de la première denture, puis ramené dans sa première position extrême, en le tournant dans l'autre sens, le deuxième bossage venant entraîner en rotation ledit organe d'entraînement, et dont ledit bras est forcé dans sa position d'entraînement, pour faire tourner ladite première couronne de comptage autour de l'axe de rotation.

Selon ce second mode de réalisation de l'invention, ledit dispositif de comptage comporte en outre un dispositif dit de course totale empêchant ledit bouton d'actionnement d'être ramené dans sa position initiale, s'il n'a pas d'abord été tourné jusqu'à son moyen d'arrêt, pour assurer un positionnement correct de ladite partie saillante dudit bras en face d'une dent de ladite première denture.

Eventuellement, ledit dispositif de course totale comporte une platine fixe solidaire dudit axe de rotation fixe et supportant un rail sensiblement annulaire s'étendant circonférentiellement par rapport audit axe de rotation sur environ 180°, et un cliquet muni d'un doigt souple, ledit cliquet étant solidaire en rotation dudit bouton d'actionnement, ledit doigt souple étant contraint, dans la position initiale du bouton d'actionnement, à l'intérieur dudit rail, ledit rail comportant une crémaillère coopérant avec ledit doigt souple pour empêcher une rotation en sens inverse dudit bouton d'actionnement, ledit doigt souple sortant dudit rail à une extrémité de celui-ci, pour permettre le retour dudit bouton d'actionnement dans sa position initiale. Avantageusement, c'est ladite extrémité dudit rail forme un moyen d'arrêt définissant la seconde position extrême dudit bouton d'actionnement.

De préférence, ladite première couronne de comptage comporte une seconde denture qui s'étend circonférentiellement par rapport audit axe de rotation fixe et dont les dents, tournées vers l'intérieur, sont réparties régulièrement autour dudit axe de rotation fixe, pour coopérer avec un premier dispositif de blocage, solidaire d'un élément tubulaire fixe monté fixement sur ledit axe de rotation fixe et comportant au moins un élément souple qui empêche ladite première couronne de comptage de tourner en sens inverse du sens de rotation imposé par ledit organe d'entraînement.

Selon le second mode de réalisation de l'invention, ladite languette d'entraînement s'étend circonférentiellement par rapport audit axe de rotation fixe, sa surface intérieur formant environ une surface annulaire, et comporte à une extrémité une tête, mobile radialement entre une position de repos, où ladite tête s'étend radialement vers l'intérieur au-delà de ladite surface annulaire intérieure, et une position d'entraînement, où ladite tête coopère avec ladite seconde couronne de comptage, ledit moyen de came étant solidaire dudit élément tubulaire fixe, et disposé sans frottement sensiblement contre ladite surface annulaire intérieure de ladite languette d'entraînement, pour forcer la tête de ladite languette dans sa position d'entraînement, chaque fois que ladite première couronne de comptage effectue un tour complet autour dudit axe de rotation

Avantageusement, ladite seconde couronne de comptage comporte une série de dents s'étendant circonférentiellement par rapport audit axe de rotation fixe et tournée vers l'intérieur, ladite série de dents étant disposée radialement à l'extérieur de ladite languette d'entraînement de ladite première couronne de comptage, de sorte que, dans sa position d'entraînement, ladite tête de la languette s'enclenche dans une dent de ladite série de dents pour entraîner ladite seconde couronne de comptage en rotation autour dudit axe de rotation fixe.

De préférence, les premières et secondes couronnes de comptage comportent des moyens d'affichage sur leurs surfaces périphériques extérieures respectives.

D'autres caractéristiques et avantages de la présente invention apparaîtront au cours de la description détaillée suivante de deux modes de réalisation donnée à titre d'exemple non limitatif en regard des dessins joints.

Sur ces dessins :
- la figure 1 est une vue schématique en coupe transversale d'un dispositif de comptage selon un premier mode de réalisation de l'invention,
- la figure 2 est une vue en coupe transversale selon une direction perpendiculaire à la coupe de la figure 1, du dispositif de comptage de la figure 1,
- la figure 3 est une vue en perspective de la première couronne de comptage du dispositif représenté sur les figures 1 et 2,
- les figures 4a et 4b sont des vues en perspective, selon deux directions différentes, de la seconde couronne de comptage du dispositif des figures 1 et 2,
- la figure 5 est une vue schématique en coupe horizontale du second dispositif anti-retour, selon le premier mode de réalisation,
- la figure 6 est une vue schématique en coupe transversale d'un dispositif de comptage selon un second mode de réalisation de l'invention,
- la figure 7 est une vue éclatée du dispositif de la figure 6,
- la figure 8 est une vue en coupe transversale du dispositif des figures 6 et 7, selon une direction perpendiculaire à la coupe de la figure 5, et représentant notamment la première couronne de comptage, et
- les figures 9a et 9b sont des vues en coupe transversale similaires à celle de la figure 8, représentant la seconde couronne de comptage respectivement dans la position de repos de la languette d'entraînement et dans sa position d'entraînement.

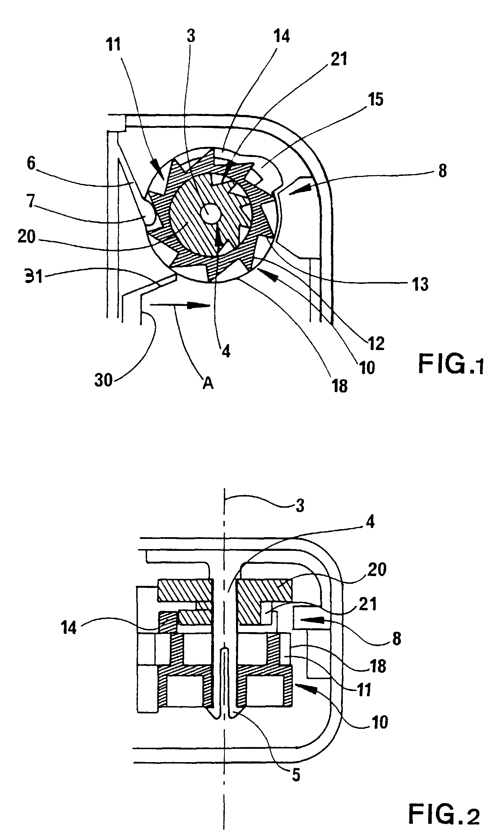

Sur les figures 1 à 5, est décrit un premier mode de réalisation du dispositif de comptage selon l'invention, qui est adapté à compter les actionnement du poussoir du distributeur de produits. Dans ce mode de réalisation, le dispositif transforme le mouvement translatif du poussoir en un mouvement rotationnel de la ou des couronnes de comptage.

Le dispositif de comptage comporte une première couronne de comptage 10 et une seconde couronne de comptage 2(). Selon l'invention, ces deux couronnes de comptage 10, 20 sont montées rotatives autour d'un même axe de rotation fixe 3, une tige 4 étant fixement montée le long dudit axe de rotation 3 pour supporter lesdites couronnes de comptage. Avantageusement, la tige fixe 4 peut comporter à son extrémité des moyens d'ancrage 5 pour empêcher tout déplacement translatif desdites couronnes de comptage 10, 20 sur ladite tige 4.

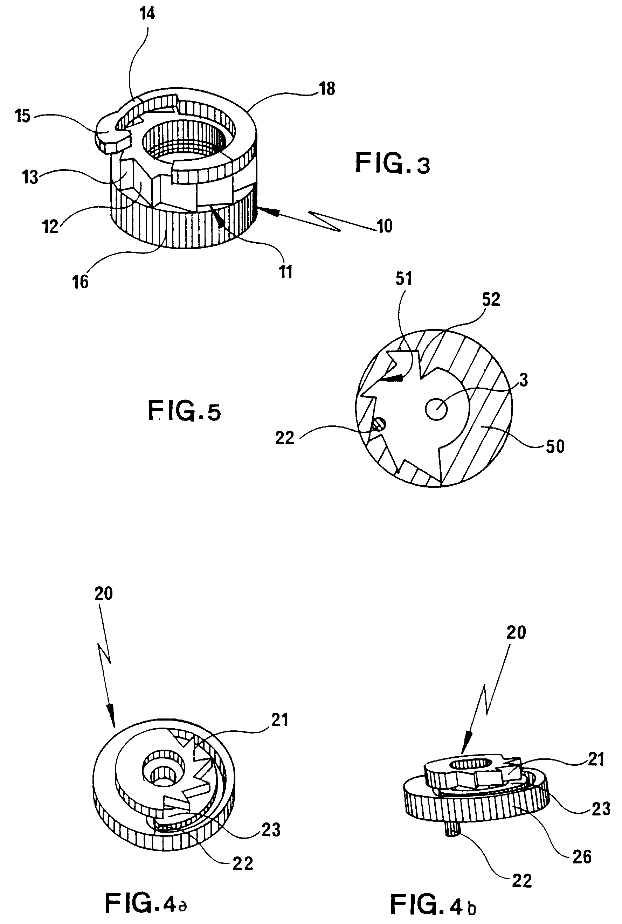

Selon l'invention, la première couronne de comptage 10 comporte une denture 11, disposée circonférentiellement par rapport audit axe de rotation 3. De préférence, comme représenté sur les figures 1 et 3, ladite couronne de comptage 10 est sensiblement annulaire et ladite denture 11 s'étend sur sa périphérie avec ses dents tournées vers l'extérieur. Ainsi, la denture 11 peut coopérer avec un organe d'entraînement 30 disposé tangentiellement à ladite première couronne 10 et qui est par exemple solidaire du poussoir du distributeur (non représenté). Cet organe d'entraînement 30 comporte avantageusement une extrémité 31 ayant une forme adaptée à venir s'enclencher dans une dent de ladite denture 11.

De préférence, ladite denture 11 comporte exactement dix dents, et la première couronne de comptage 10 agit donc comme compteur des unités.

Comme représenté sur le figures 1 et 3, les dents de ladite denture 11 sont toutes identiques et comportent une paroi de fond inclinée 12 s'étendant environ circonférentiellement par rapport à l'axe de rotation 3 et une paroi de butée 13, environ perpendiculaire à ladite paroi de fond 12 et s'étendant donc environ radialement par rapport audit axe 3. En fonctionnement, l'extrémité 31 de l'organe d'entraînement 30 s'engage le long de ladite paroi de fond 12 suivant la flèche A de la figure 1 pour venir buter contre ladite paroi de butée 13, et ainsi entraîner la première couronne de comptage 10 en rotation autour de l'axe de rotation 3, en exerçant une poussée sur ladite paroi de butée 13. Lorsque le poussoir (non représenté), et donc l'organe d'entraînement 30 reviennent dans leur position de repos, l'extrémité 31 dudit organe d'entraînement glisse le long de ladite paroi de fond 12 dans le sens opposé à la flèche A pour venir se placer en face de la dent suivante de ladite denture 11. Avantageusement, l'extrémité 31 présente une certaine élasticité pour ne pas opposer une forte résistance, par exemple par frottements, lors du retour de l'organe d'entraînement 30 dans sa position de repos. De préférence, l'organe d'entraînement 30 est disposé de telle manière à ce qu'à chaque actionnement, il fait tourner la première couronne de comptage 10 d'une distance angulaire correspondant à exactement une dent de la denture 11.

Afin d'éviter que, lors du retour de l'organe d'entraînement 30 dans sa position de repos, la première couronne de comptage 10 ne soit entraînée en rotation du fait d'un éventuel frottement de ladite extrémité 31 de l'organe d'entraînement 30 sur la paroi de fond 12 d'une dent de la denture 111, il est prévu un premier dispositif de blocage 6 agissant sur la denture 11.

Ce dispositif de blocage comporte avantageusement une lame souple 6, fixe par rapport audit axe de rotation 3, et dont l'extrémité 7 vient s'encliqueter dans le denture 11. En raison de sa souplesse, ladite laine 6 peut, lorsque la couronne 10 est entraînée en rotation par l'organe d'entraînement 3(), glisser sur la paroi de fond 12 de la dent avec laquelle elle coopère et venir s'encliqueter dans la dent suivante.

Cet encliquetage peut présenter plusieurs avantages.

En premier lieu, de par la souplesse de la lame, il provoque un léger bruit qui indique utilement à l'utilisateur que la couronne de comptage 10 a avancé d'une unité. D'autre part, dans les inhalateurs à poudre où la dose de produit tombe dans la chambre de dosage par gravité, l'encliquetage de la lame 6 provoque de légères vibrations qui peuvent favoriser le remplissage de ladite chambre de dosage.

Bien entendu, comme cela apparaît clairement sur la figure 1, l'extrémité 7 de la lame 6 empêche la couronne de comptage 10 de tourner en sens inverse du sens de rotation imposé par l'organe d'entraînement 30, en venant s'appuyer contre la paroi de butée 13 de la dent correspondante.

La première couronne de comptage 10 comporte en outre une languette d'entraînement 14, destinée à entraîner en rotation la seconde couronne de comptage 20. Cette languette 14 s'étend de préférence circonférentiellement par rapport audit axe de rotation fixe 3 et est mobile radialement entre une position de repos et une position d'entraînement. Avantageusement, cette mobilité est assurée par une certaine souplesse de ladite languette 14.

Comme représenté sur la figure 3, la languette d'entraînement s'étend sur une partie de la périphérie de la première couronne de comptage 10 et comporte à une des ses extrémités une tête 15. Dans la position de repos de la languette 14, sa tête 15 s'étend radialement vers l'extérieur au-delà de la surface annulaire extérieure 18 de ladite première couronne de comptage 10, et ne coopère pas avec la seconde couronne de comptage 20. Dans la position d'entraînement de la languette 14, sa tête 15 coopère avec ladite seconde couronne 20 pour l'entraîner en rotation autour dudit axe de rotation 3.

Selon l'invention, la languette 14 est forcée dans sa position d'entraînement par un moyen de came 8. Ce moyen de came 8 est de préférence fixe par rapport à l'axe de rotation 3 et peut avantageusement être solidaire de la tige 4 qui supporte les deux couronnes de comptage 10 et 20. Il est disposé sans frottement sensiblement contre ladite surface annulaire extérieure 18 de la première couronne de comptage, et notamment au niveau de la languette d'entraînement 14. Ainsi, chaque fois que la première couronne de comptage effectue un tour complet autour de l'axe de rotation 3, la tête 15 de la languette passe devant ledit moyen de came 8 et est forcé dans sa position d'entraînement. Dans l'exemple de réalisation représenté sur les figures 1 et 2, le moyen de came 8 comporte du côté en regard de la languette 14, un profil arrondi adapté à la surface annulaire extérieure 18 de la première couronne de comptage 10, ledit profil s'étendant sur une longueur correspondant environ à la longueur angulaire d'une dent de la denture 11. La tête 15 de la languette d'entraînement 14 présente sur son côté extérieur un profil complémentaire à celui dudit moyen de came 8, et sur son côté intérieur, un profil complémentaire à celui des dents de la denture 11. Il est ainsi assuré que la languette d'entraînement 14 n'est forcée dans sa position d'entraînement qu'une seule fois sur un tour complet de la première couronne de comptage 10, et seulement sur une distance angulaire correspondant à une des dix dents de la denture 11. La première couronne de comptage 10 remplit ainsi parfaitement sa fonction de compteur des unités.

La seconde couronne de comptage 20 est représentée en détail sur les figures 4a et 4b. Elle est de forme générale sensiblement annulaire et vient comme la première couronne 10, s'enfiler sur la tige fixe 4 autour de laquelle elle peut tourner, mais le long de laquelle elle ne peut pas coulisser en translation. Selon l'invention, cette seconde couronne de comptage 20 comporte une série de dents 21 disposée circonférentiellement par rapport à l'axe de rotation 3. Avantageusement, cette série de dents 21 est décalée radialement vers l'intérieur en direction de l'axe de rotation 3, de sorte que, lorsque montée sur ladite tige fixe 4, ladite série de dents 21 de la seconde couronne de comptage 20 est disposée radialement à l'intérieur de ladite languette d'entraînement 14 de la première couronne de comptage. De préférence, les deux couronnes tournent l'une par rapport à l'autre avec un faible frottement. Ainsi, lorsque la tête 15 de la languette d'entraînement 14 est forcée radialement dans sa position d'entraînement, elle vient en prise avec une dent de ladite série de dents 21 de la seconde couronne de comptage 20 pour l'entraîner en rotation. Avantageusement, les dents de ladite série de dents 21 ont une forme environ similaire à celle de la denture 11 de la première couronne 10, pour assurer une coopération efficace avec la tête 15 de la languette 14. De préférence, l'écart angulaire entre deux dents successives de ladite série de dents 21 de la seconde couronne 20 est identique à l'écart angulaire entre deux dents successives de la denture 11 de la première couronne 10. Ainsi, la languette d'entraînement 14 dans sa position d'entraînement fait avancer la seconde couronne de comptage 20 d'exactement une dent à chaque tour. Cette seconde couronne 20 agit donc comme compteur des dizaines lorsque la première couronne 10 agit comme compteur des unités.

Afin d'éviter que la seconde couronne de comptage 20 ne tourne en sens inverse du sens de rotation imposé par ladite languette d'entraînement 14 de la première couronne de comptage 10, il est prévu un second dispositif de blocage. Avantageusement, ce second dispositif de blocage comporte une platine de blocage 50 fixe par rapport à l'axe de rotation 3, ladite platine de blocage 50 étant destinée à coopérer avec un ergot 22 solidaire de la seconde couronne de comptage 2(). De préférence, ladite platine de de blocage 50 supporte un profil cannelé 51 dont les cannelures s'étendent circonférentiellement par rapport audit axe de rotation 3. Comme représenté sur la figure 5, le profil cannelé 51 peut être tourné vers l'intérieur, mais il pourrait également être tourné vers l'extérieur. En outre, les cannelures représentées sur les figures 5 ont une forme semblable à une dent mais elle peuvent avoir toute forme adaptée à retenir de manière non fixe ledit ergot 22 de la seconde couronne de comptage 20. Ainsi, le profil cannelé 51 empêche non seulement une rotation de la seconde couronne de comptage 20 en sens inverse du sens de rotation imposé par la languette 14, mais il empêche également toute rotation dans le sens de rotation imposé par la languette 14, lorsque ladite languette est dans sa position de repos. Les frottements pouvant exister entre les première et seconde couronnes de comptage 10 et 20 n'entraînent donc pas une rotation de la seconde couronne de comptage 20. Avantageusement, ledit profil cannelé 51 comporte un nombre de cannelures identique au nombre de dents de la série de dents 21 de la seconde couronne de comptage 20. Il peut en outre comporter à son extrémité correspondant à la dernière cannelure, un moyen de butée 52 bloquant ledit ergot 22 en rotation et empêchant par conséquent la seconde couronne 20 de poursuivre sa rotation. La seconde couronne 20 ne pouvant plus tourner, elle bloque la première couronne 10 au moment où la languette 14 est forcée dans sa position d'entraînement. Si, comme cela est préférable, l'actionnement du distributeur de produit est lié au comptage de la dose à distribuer, ledit moyen de butée 52 bloque donc également l'actionnement dudit distributeur. Le nombre maximal de doses émises par ce distributeur est donc déterminé par le nombre de cannelures dudit profil cannelé 51 qui sont situées avant ledit moyen de butée 52 (dans le sens de rotation correspondant au comptage). L'ergot 22, solidaire de la seconde couronne de comptage 20, est de préférence fixé à l'extrémité d'une patte souple 23 de ladite seconde couronne, qui s'étend circonférentiellement par rapport à l'axe de rotation 3. L'ergot 22 peut donc, en raison de la souplesse radiale de la patte 23, être contraint vers la cannelure suivante du profil cannelé 51, lorsque la seconde couronne 20 est entraînée en rotation par la languette d'entraînement 14 de la première couronne 10.

De préférence, les première et seconde couronnes de comptage comportent des moyens d'affichage tels que des chiffres permettant d'indiquer à l'utilisateur, soit le nombre de doses émises, soit le nombre de doses restant à émettre. Avantageusement, cet affichage est situé sur les surfaces périphériques extérieures 16 et 26 des premières et secondes couronnes de comptage 10 et 20, respectivement. Ainsi, la première couronne de comptage 10 comporte les chiffres de 0 à 9 répartis sur sa périphérie, chaque chiffre correspondant à une dent de la denture 11. Dans l'exemple représenté sur les figures 1 à 5, la seconde couronne de comptage comporte cinq dents, le nombre maximal de doses émises par le distributeur est donc de quarante-neuf. Il est bien sûr possible de déterminer un nombre maximal de doses différent, en prévoyant un nombre de dents différent sur la seconde couronne de comptage.

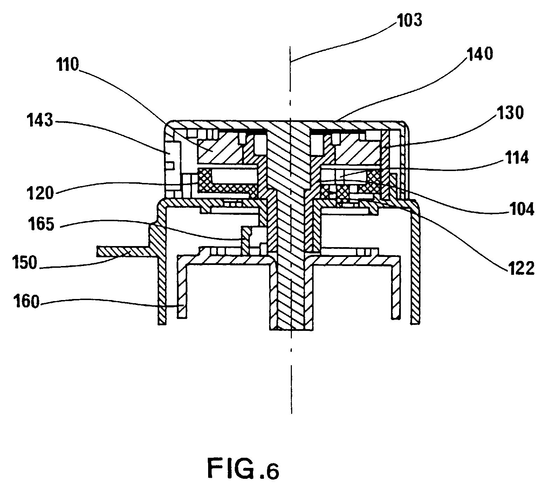

Sur les figures 6 à 9 est représenté un second mode de réalisation de l'invention. Ce second mode de réalisation concerne un dispositif de comptage qui est actionné au moyen d'un organe d'entraînement exerçant un mouvement rotationnel. Par exemple, il existe des inhalateurs comprenant un mécanisme à air comprimé pour expulser les doses de produit hors de la chambre de dosage, ladite chambre de dosage étant remplie par des moyens de remplissage rotationnels. Le remplissage de la chambre de dosage n'est dans ce cas pas directement lié à l'actionnement du mécanisme d'expulsion.

Le mécanisme d'expulsion n'étant actionnable qu'après un remplissage effectif de la chambre de dosage, il est avantageux dans ce genre de distributeur de compter lesdits remplissages de la chambre de dosage.

Le dispositif de comptage selon ce second mode de réalisation de l'invention comporte une première et une seconde couronnes de comptage 110, 120, montées librement en rotation autour d'un axe de rotation fixe 103.

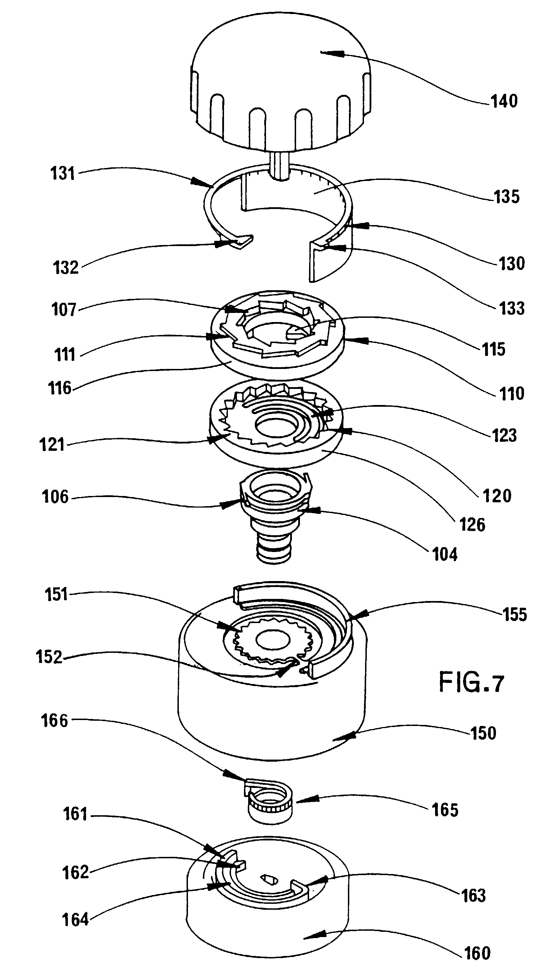

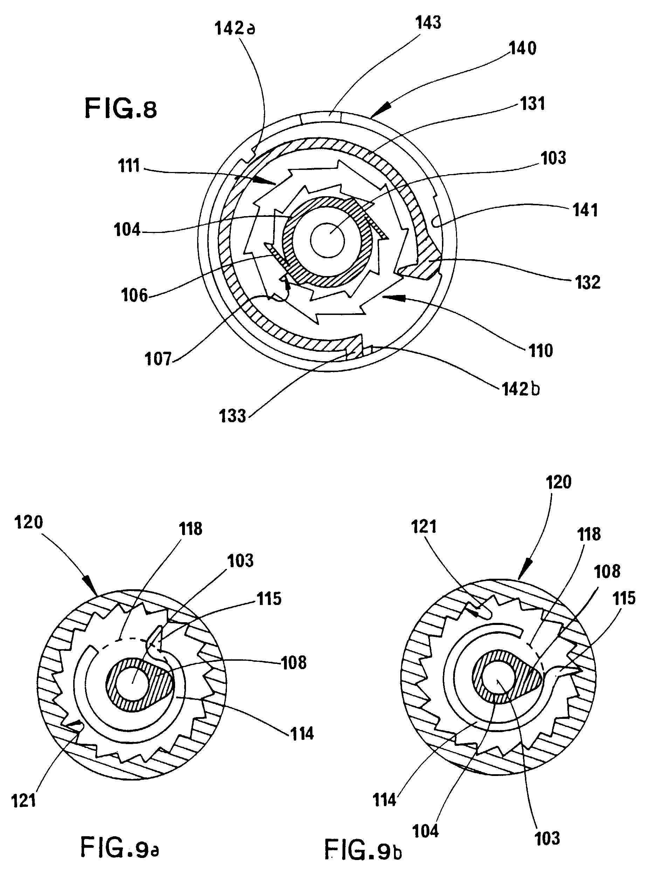

Avantageusement, un élément tubulaire fixe 104 est monté le long dudit axe de rotation 103 pour supporter les deux couronnes de comptage 110, 120 librement en rotation. Comme décrit précédemment pour le premier mode de réalisation, la première couronne de comptage 110 est entraînée en rotation autour de l'axe de rotation fixe 103 par un organe d'entraînement 130, qui coopère avec une première denture 111 de ladite première couronne 110. Cette première de denture 111 s'étend circonférentiellement par rapport audit axe de rotation 103 et comporte exactement dix dents lorsque la première couronne 110 agit comme compteur des unités. Avantageusement, les dents de cette première denture 111 sont toutes identiques, réparties régulièrement sur la totalité de la circonférence, et sont tournées vers l'extérieur. L'organe d'entraînement 130 est annulaire et est monté rotatif autour dudit axe de rotation 103, de manière à entourer ladite première couronne de comptage 110. Il comporte un bras souple annulaire 131 s'étendant circonférentiellement par rapport audit axe de rotation 103, mobile radialement entre une position de repos et une position d'entraînement, ledit bras 131 étant solidaire d'un élément de paroi annulaire 135 qui s'étend avantageusement sur environ la moitié de la circonférence de l'organe d'entraînement 130. Dans sa position de repos où le bras 131 de l'organe d'entraînement 130 ne coopère pas avec la denture 111 de la première couronne 110, une partie saillante 132 dudit bras s'étend au-delà de la surface annulaire extérieure formée par la paroi extérieure dudit bras 131. Avantageusement, cette partie saillante 132 correspond à une extrémité du bras 131, comme représenté sur les figures 7 et 8. Dans sa position d'entraînement, ladite partie saillante 132 coopère avec la denture 111 de la première couronne 110 pour entraîner celle-ci en rotation autour de l'axe de rotation 103.

Le dispositif comporte en outre un bouton d'actionnement 140 manipulé par l'utilisateur. Ce bouton d'actionnement sert par exemple au remplissage de la chambre de dosage du distributeur, comme décrit ci-dessus. Selon le présent mode de réalisation de l'invention, le bouton d'actionnement 140 est également annulaire et monté rotatif autour de l'axe de rotation 103, de manière à entourer l'organe d'entraînement 130, et notamment son bras souple 131. Il comporte des moyens 142 pour faire tourner ledit organe d'entraînememnt 130 autour dudit axe de rotation 103 et des moyens 141 pour forcer ledit bras 131 dudit organe d'entraînement 130 dans sa position d'entraînement. Il comporte en outre une fenêtre 143 dans sa paroi latérale permettant à l'utilisateur de visualiser le nombre de doses émises ou restant à émettre. Comme pour le premier mode de réalisation décrit précédemment, l'affichage est avantageusement situé sur les surfaces périphériques extérieures 116 et 126 des première et seconde couronnes de comptage 110 et 120, respectivement.

Avantageusement, lesdits moyens pour faire tourner l'organe d'entraînement comportent deux bossages 142a, 142b, situés sur la face annulaire interne du bouton d'actionnement 140. Ils coopèrent avec ledit organe d'entraînement, par exemple par l'intermédiaire du bras souple 131. De même, les moyens pour forcer le bras 131 dans sa position d'entraînement sont également disposés sur la face interne du bouton d'actionnement 140 et peuvent par exemple être réalisés sous la forme d'un renflement 141 qui se projète vers l'intérieur dudit bouton d'actionnement.

Le fonctionnement du dispositif est le suivant.

Dans la position de repos du dispositif de comptage, l'ensemble formé du bouton d'actionnement 140, de l'organe d'entraînement 130 et de la première couronne de comptage 110 est dans une position correspondant à la position finale de la procédure de comptage de la dose précédente. Ainsi, la partie saillante 132 du bras souple 131, par exemple une première extrémité dudit bras, est forcée dans sa position d'entraînement par le renflement 141, le bouton d'actionnement 140, mobile entre deux positions extrêmes, séparées de préférence d'une distance correspondant à une rotation d'environ 180°, étant dans sa première position extrême, dans laquelle il ne peut tourner que dans un sens, par exemple le sens trigonométrique direct comme représenté sur la figure 8. Le second bossage 142b est en contact avec la seconde extrémité 133 du bras 131 et le premier bossage 142a est avantageusement séparé du second bossage 142b par une distance correspondant à un angle d'environ 144°.

Ainsi, lorsque l'utilisateur actionne le dispositif, il tourne le bouton d'actionnement 140 dans le sens direct. Le renflement 141 n'est plus en contact avec la partie saillante 132 du bras souple 131, et ledit bras reprend donc par élasticité sa position de repos où il ne coopère pas avec la première couronne de comptage 110. Simultanément, le second bossage 142b se dégage de la seconde extrémité 133 du bras 131. Après une rotation d'environ 144°, le première bossage 142 vient s'appuyer sur avec ladite seconde extrémité 133 du bras 131. Une poursuite de la rotation du bouton d'actionnement 140 provoque donc la rotation de l'organe d'entraînement 130. La rotation maximale du bouton d'actionnement 140 étant d'environ 180°, l'organe d'entraînement 130 toujours dans sa position de repos tourne donc d'une distance correspondant à un angle d'environ 36°, ce qui correspond exactement à une dent de la denture 111 de la première couronne de comptage 110. En bout de course du bouton d'actionnement 140, la partie saillante 132 du bras 131 de l'organe d'entraînement est donc positionnée face à la prochaine dent de la denture 111. Lorsque le bouton d'actionnement arrive dans sa seconde position extrême, après une rotation d'environ 180°, l'utilisateur le ramène dans sa position initiale en le tournant en sens inverse, c'est-à-dire dans le sens trigonométrique indirect dans l'exemple représenté sur la figure 8. A nouveau, après une rotation de - 144°, le second bossage 142b vient buter sur l'extrémitré 133 du bras souple 131 et simultanément, le renflement 141 coopère avec la partie saillante 132 dudit bras souple pour forcer ce dernier dans Sa position d'entraînement. La partie saillante 132 est donc en prise avec une dent de la denture 111 et une poursuite de la rotation du bouton d'actionnement 140 provoque la rotation de ladite première couronne de comptage 110. Après une rotation de - 180° du bouton d'actionnement 140, à partir de sa seconde position extrême, ledit bouton d'actionnement retrouve sa première position extrême et la procèdure de comptage est terminée.

La première couronne de comptage a ainsi tourné autour de l'axe de rotation 103 d'un angle de -36° environ, ce qui correspond à l'écartement entre deux dents successives de la denture 111.

Avantageusement, on prévoit un dispositif dit de course total pour empêcher le bouton d'actionnement 140 d'être ramené dans sa première position extrême avant qu'il n'ait atteint sa seconde position extrême. On assure ainsi un positionnement précis de la partie saillante 132 du bras 131 devant la dent suivante de la denture 111. Ce dispositif comporte avantageusement une platine 160 fixe par rapport à l'axe de rotation 103 et un cliquet 165 solidaire en rotation du bouton d'actionnement 140, c'est-à-dire angulairement dépendant en rotation de celui-ci. La platine 160 supporte un rail 161 qui s'étend circonférentiellement par rapport audit axe de rotation 103 sur environ 180°. Ce rail 161 comporte à son entrée une partie de paroi intérieure évasée 162, à sa sortie une paroi de butée 163, et entre ses deux extrémités, une crémallière 164 orientée axialement. Le cliquet 165 comporte un doigt souple 166 qui, dans la position de repos du dispositif, c'est-à-dire la première position extrême du bouton d'actionnement 140, est disposé à l'intérieur de la partie de paroi évasée 162 du rail 161.

Lorsque le bouton d'actionnement 140 est tourné, ledit doigt souple 166 est contraint dans le rail 161 par la partie de paroi évasée 162 et vient coopérer avec ladite crémaillère 164. Les dents de cette crémallière 164 sont telles que le doigt souple 166 peut passer d'une dent à l'autre lorsque le bouton d'actionnement 140 est tourné en direction de sa seconde position extrême, mais ne peut pas passer d'une dent à l'autre lorsqu'il est tourné en direction de sa première position extrême. Il n'est donc pas possible de ramener le bouton d'actionnement 140 dans sa position initiale avant d'avoir atteint la sortie 163 du rail 161. A la sortie du rail, le doigt souple 166 se désengage de la crémaillère et reprend par élasticité sa position non contrainte hors du rail 161. Le bouton d'actionnement 140 peut alors être ramené dans sa première position extrême, pour compléter la procédure de comptage. Eventuellement, on peut prévoir un moyen de rappel automatique, tel qu'un ressort par exemple, pour ramener automatiquement le bouton d'actionnement dans sa première position extrême.

D'autre part, on prévoit un premier dispositif de blocage pour empêcher la première couronne de comptage 110 de tourner en sens inverse du sens de rotation imposé par l'organe d'entraînement 130. Ce premier dispositif de blocage comporte avantageusement au moins un élément souple 106, solidaire de l'élément tubulaire fixe 104, et qui vient en prise avec une seconde denture 107 de la première couronne de comptage 110. Cette seconde denture 107 est concentrique à la première denture 111, mais ses dents sont tournées vers l'intérieur pour coopérer avec l'élément souple 106.

L'intercation entre la première et la seconde couronne de comptage est sensiblement identique à celle du premier mode de réalisation de la l'invention décrit précédemment. La seule différence est que la série de dents 121 de la seconde couronne de comptage 120 a ses dents tournées vers l'intérieur, et la languette d'entraînement 114 de la première couronne de comptage 140 est forcée dans sa position d'entraînement, où sa tête 115 vient en prise avec une dent de ladite série de dents 121, par un moyen de came 108 fixe par rapport à l'axe de rotation 103 et solidaire de l'élément tubulaire fixe 104. Ainsi, la tête 115 de languette 114 s'étend, dans sa position de repos, radialement vers l'intérieur au-delà de la surface annulaire intérieure 118 de ladite languette 114, et ledit moyen de came 108 est disposé sans frottement sensiblement contre ladite surface annulaire intérieure 118. Lorsque la tête 115 passe au niveau du moyen de came 108, celui-ci la force donc radialement vers l'extérieur pour la mettre en prise avec la série de dents 121 de la seconde couronne de comptage 120.

Comme dans le premier mode de réalisation, la seconde couronne de comptage 120 comporte un second dispositif de blocage. Ce second dispositif de blocage comporte également un profil cannelé 151 supporté par un couvercle 150, fixe par rapport à l'axe de rotation 103 qui coopère ave un ergot 122 solidaire d'une patte souple 123 de ladite seconde couronne 120. Le fonctionnement de ce second disposiotif de blocage est similaire à celui décrit précédemment en relation au premier mode de réalisation.

Dans l'exemple représenté sur les figures 7 et 9, la seconde couronne de comptage 120 et le profil cannelé 151 comporte respectivement vingt dents et cannelures. Ce compteur est donc adapté à compter 199 doses de produit. Avantageusement, le couvercle 150 peut également supporter un guidage 155 du bouton d'actionnement 140, et de l'élément de paroi 135 de l'organe d'entraînement 130, ledit guidage définissant les deux positions extrêmes dudit bouton 140.

Ce second mode de réalisation de l'invention a été décrit en référence aux figures 6 à 9 représentant un exemple de réalisation. Il est clair que le dispositif fonctionne également avec un bouton d'actionnement effectuant plus ou moins d'un demi-tour entre ses deux positions extrêmes. Il suffit alors d'adapter l'écart des deux bossages 142a, 142b ainsi que le positionnement du renflement 141 pour obtenir le même résultat. De même, on peut imaginer le dispositif fonctionnant avec des sens de rotation inversés.

De préférence, les première et seconde couronnes de comptage (10, 110 ; 20, 120) sont réalisées en une pièce à partir de matières plastiques résistantes, ce qui leur procure une grande solidité et fiabilité, et élimine le risque de déterrioration, notamment des parties souples et élastiques.

## Revendications

1. Dispositif de comptage des doses de produit émises par un distributeur de produits fluides ou pulvérulents, comprenant une première couronne de comptage (10, 110) et une seconde couronne de comptage (20, 120), les deux couronnes de comptage étant montées rotatives autour d'un axe de rotation fixe (3, 103), ladite première couronne de comptage (10, 110) comportant une denture (11, 111), disposée circonférentiellement par rapport audit axe de rotation fixe (3, 103), coopérant avec un organe d'entraînement (30, 130) destiné à faire tourner ladite première couronne de comptage autour dudit axe de rotation fixe à chaque utilisation du distributeur, ladite première couronne de comptage (10, 110) comportant en outre une languette d'entraînement (14, 114) mobile entre une position de repos, où elle ne coopère pas avec ladite seconde couronne de comptage (20, 120), et une position d'entraînement, où elle coopère avec ladite seconde couronne de comptage (20, 120) pour la faire tourner autour dudit axe de rotation fixe, ladite languette d'entraînement (14, 114) étant forcée dans sa position d'entraînement par un moyen de came (8, 108), caractérisé en ce que ladite seconde couronne de comptage (20, 120) coopère, après l'émission d'un nombre prédéterminé de doses, avec un moyen de butée fixe (52, 152) qui empêche toute rotation ultérieure de ladite seconde couronne de comptage (20, 120).

2. Dispositif de comptage selon la revendication 1, dans lequel ladite seconde couronne de comptage (20, 120) comporte une série de dents (21, 121) disposées circonférentiellement par rapport audit axe de rotation fixe (3, 103) et ladite languette d'entraînement (14, 114) de ladite première couronne de comptage (10, 110) comporte à une extrémité une tête (15, 115), ladite tête venant s'enclencher dans ladite série de dents (21, 121) de ladite seconde couronne de comptage lorsque ladite languette d'entraînement est dans sa position d'entraînement.

3. Dispositif de comptage selon la revendication 1 ou la revendication 2, dans lequel ladite première couronne de comptage (10, 110), agissant comme compteur d'unité comporte une denture périphérique (11, 111) contenant dix dents, lesdites dix dents étant réparties régulièrement autour dudit axe fixe (3, 103), ladite languette d'entraînement (14, 114) coopérant avec ledit moyen de came (8, 108) pour entraîner en rotation ladite seconde couronne de comptage (20, 120), agissant comme compteur de dizaines, chaque fois que ladite première couronne de comptage effectue un tour complet autour dudit axe de rotation fixe.

4. Dispositif de comptage selon l'une quelconque des revendications précédentes, dans lequel sont prévus un premier dispositif de blocage (6, 106) agissant sur la première couronne de comptage (10, 110) pour l'empêcher de tourner en sens inverse du sens de rotation imposé par ledit élément d'entraînement (30, 130), et un second dispositif de blocage (23, 51 ; 123, 151) agissant sur ladite seconde couronne de comptage (20, 120) pour l'empêcher de tourner en sens inverse du sens de rotation imposé par ladite languette d'entraînement (14, 114) de ladite première couronne de comptage (10, 110).

5. Dispositif de comptage selon la revendication 4, dans lequel ledit second dispositif de blocage comprend une patte souple (23, 123) solidaire de ladite seconde couronne de comptage, et munie à une de ses extrémités d'un ergot (22, 122), ledit ergot coopérant avec un profil cannelé (51, 151), fixe par rapport à l'axe de rotation (103), pour empêcher ladite second couronne de comptage de tourner dans un sens quelconque lorsque ladite languette d'entraînement (14, 114) de ladite première couronne de comptage est dans sa position de repos.

6. Dispositif de comptage selon la revendication 5, dans lequel ledit profil cannelé fixe (51, 151) qui coopère avec ledit ergot (22, 122) de ladite patte souple (23, 123) dudit second dispositif de blocage comporte ledit moyen de butée (52, 152) fixe bloquant ledit ergot (22, 122) de ladite patte souple, empêchant ainsi la rotation de ladite seconde couronne de comptage, le nombre maximal de doses émises par le distributeur étant ainsi déterminé par le nombre de cannelures du profil cannelé situées avant ledit moyen de butée (52, 152).

7. Dispositif de comptage selon l'une quelconque des revendications précédentes, dans lequel une tige (4) est fixement montée sur ledit axe de rotation fixe (3) et les première et seconde couronnes de comptages (10, 20), sensiblement annulaires, sont montées rotatives sur ladite tige fixe (4), ladite première couronne de comptage (10) comportant une denture périphérique (11) qui s'étend circonférentiellement par rapport audit axe fixe (3) et dont les dents sont tournées vers l'extérieur, ladite denture (11) coopérant avec un organe d'entraînement (30) solidaire d'un poussoir d'actionnement du distributeur et exerçant un mouvement translatif, ledit élément d'entraînement (30) coopérant à chaque actionnement du poussoir avec une dent de ladite denture (11) pour faire tourner ladite première couronne de comptage (10) autour dudit axe de rotation fixe (3).

8. Dispositif de comptage selon la revendication 7, dans lequel est prévu un premier dispositif de blocage comportant une lame souple fixe (6) qui coopère avec la denture (11) de ladite première couronne de comptage (10) pour empêcher celle-ci de tourner en sens inverse du sens de rotation imposé par ledit organe d'entraînement (30).

9. Dispositif de comptage selon la revendication 7 ou la revendication 8, dans lequel ladite première couronne de comptage (10) comporte une languette d'entraînement (14) qui s'étend circonférentiellement par rapport audit axe de rotation fixe (3) et comporte à une extrémité une tête (15), mobile radialement entre une position de repos, où ladite tête (15) s'étend radialement vers l'extérieur au-delà de la surface annulaire extérieure (18) de la première couronne de comptage (10), et une position d'entraînement, où ladite tête (15) coopère avec ladite seconde couronne de comptage (20), ledit moyen de came (8) étant fixe par rapport audit axe de roattion (3) et disposé sans frottement sensiblement contre ladite surface annulaire extérieure (18) de ladite première couronne de comptage (10) au niveau de ladite languette d'entraînement (14), pour forcer la tête (15) de ladite languette d'entraînement (14) dans sa position d'entraînement chaque fois que ladite première couronne de comptage (10) effectue un tour complet autour dudit axe de rotation fixe (3).

10. Dispositif de comptage selon la revendication 9, dans lequel ladite seconde couronne de comptage (20) comporte une série de dents s'étendant circonférentiellement par rapport audit axe de rotation fixe (3) et tournées vers l'extérieur, ladite série de dents étant disposée radialement à l'intérieur de ladite languette d'entraînement (14) de ladite première couronne de comptage, de sorte que dans sa position d'entraînement, ladite tête (5) de la languette (14) s'enclenche dans une dent de ladite série de dents pour entraîner ladite seconde couronne de comptage (20) en rotation autour dudit axe de rotation fixe (3).

11. Dispositif de comptage selon la revendication 10, dans lequel le côté extérieur de ladite tête (15) de la languette d'entraînement (14) a un profil complémentaire au profil dudit moyen de came fixe (8), et le côté intérieur de ladite tête (15) a un profil complémentaire au profil desdites dents de ladite série de dents de la seconde couronne de comptage (20).

12. Dispositif de comptage selon l'une quelconque des revendications 1 à 6, dans lequel ladite première couronne de comptage (110) comporte une première denture (111) qui s'étend circonférentiellement par rapport audit axe de rotation fixe (103) et dont les dents, tournées vers l'extérieur, sont réparties régulièrement autour dudit axe fixe (103), pour coopérer avec un organe d'entraînement (130) exerçant un mouvement rotatif, ledit organe d'entraînement (130), étant actionné par l'utilisateur au moyen d'un bouton d'actionnement mobile en rotation autour de l'axe de rotation (103) entre une première et une seconde positions extrêmes.

13. Dispositif de comptage selon la revendication 12, dans lequel ledit organe d'entraînement (130) est annulaire, et monté rotatif autour dudit axe de rotation fixe (103) et comporte un bras souple annulaire (131), mobile radialement entre une position de repos, où une partie saillante (132) dudit bras (131) s'étend radialement vers l'extérieur au-delà de la surface extérieure annulaire dudit bras (131), et une position d'entraînement, où ladite partie saillante (132) du bras (131) coopère avec une dent de ladite première denture (111) de ladite première couronne de comptage (110) pour l'entraîner en rotation, ledit bras souple (131) étant forcé dans sa position d'entraînement par le bouton d'actionnement (140).

14. Dispositif de comptage selon la revendication 13, dans lequel ledit bouton d'actionnement (140) est annulaire et monté rotatif autour dudit axe de rotation fixe (103) de manière à entourer ledit organe d'entraînement, (130), ledit bouton d'actionnement (140) comportant des moyens (142) pour faire tourner ledit organe d'entraînement (130) autour dudit axe de rotation fixe (103) et des moyens (141) pour forcer ledit bras (131) dans sa position d'entraînement.

15. Dispositif de comptage selon la revendication 14, dans lequel lesdits moyens pour forcer ledit bras (131) dans sa position d'entraînement comportent un renflement (141) disposé sur la face annulaire interne dudit bouton d'actionnement (140), et lesdits moyens pour faire tourner l'organe d'entraînement (131) comportent deux bossages (142a, 142b) qui coopèrent avec ledit organe d'entraînement (130), les deux bossages (142a, 142b) étant disposés à même hauteur sur la face annulaire interne dudit bouton d'actionnement, (140) le premier bossage étant adapté à faire tourner l'organe d'entraînement (131) dans un sens pour amener la partie saillante (132) de son bras (131) en face d'une dent de ladite première denture (111), et le deuxième bossage (142b) étant adapté à entraîner l'organe d'entraînement (130) dans l'autre sens pour faire tourner ladite première couronne de comptage (110), lorsque le bras souple (131) est dans sa position d'entraînement.

16. Dispositif de comptage selon la revendication 15, dans lequel ladite première denture (111) de ladite première couronne de comptage (110) comporte dix dents, la distance angulaire entre les deux positions extrêmes du bouton d'actionnement (140) est d'environ 180°, et lesdits bossages (142a, 142b) sont disposés à un espacement angulaire d'environ 144° l'un de l'autre, ledit bouton d'actionnement (140) étant d'abord tourné de 180° dans un sens vers sa seconde position extrême pour amener ladite partie saillante (132) du bras (131) de l'organe d'entraînement (130) en face de la dent suivante de la première denture (111), puis ramené dans sa première position extrême, en le tournant dans l'autre sens, le deuxième bossage (142b) venant entraîner en rotation ledit organe d'entraînement (130), et dont ledit bras (131) est forcé dans sa position d'entraînement, pour faire tourner ladite première couronne de comptage (110) autour de l'axe de rotation (103).

17. Dispositif de comptage selon la revendication 16, dans lequel ledit dispositif de comptage comporte en outre un dispositif dit de course totale empêchant ledit bouton d'actionnement (140) d'être ramené dans sa position initiale s'il n'a pas d'abord été tourné jusqu'à son moyen d'arrêt, pour assurer un positionnement correct de ladite partie saillante (132) dudit bras (131) en face d'une dent de ladite première denture (111).

18. Dispositif de comptage selon la revendication 17, dans lequel ledit dispositif de course totale comporte une platine fixe (160) solidaire dudit axe de rotation fixe (103) et supportant un rail (161) sensiblement annulaire s'étendant circonférentiellement par rapport audit axe de rotation (103) sur environ 180°, et un cliquet (165) muni d'un doigt souple (166), ledit cliquet (165) étant solidaire en rotation dudit bouton d'actionnement (140), ledit doigt souple (166) étant contraint, dans la position initiale du bouton d'actionnement, à l'intérieur dudit rail, ledit rail (161) comportant une crémaillère (164) coopérant avec ledit doigt souple (166) pour empêcher une rotation en sens inverse dudit bouton d'actionnement (140), ledit doigt souple (166) sortant dudit rail (161) à une extrémité (163) de celui-ci, pour permettre le retour dudit bouton d'actionnement (140) dans sa position initiale.

19. Dispositif de comptage selon la revendication 18, dans lequel ladite extrémité (163) dudit rail (161) forme un moyen d'arrêt définissant la seconde position extrême dudit bouton d'actionnement.

20. Dispositif de comptage selon l'une quelconque des revendications 12 à 19, dans lequel ladite première couronne de comptage (110) comporte une seconde denture (107) qui s'étend circonférentiellement par rapport audit axe de rotation fixe (103) et dont les dents, tournées vers l'intérieur, sont réparties régulièrement autour dudit axe de rotation fixe (103), pour coopérer avec un premier dispositif de blocage, solidaire d'un élément tubulaire fixe (104) monté fixement sur ledit axe de rotation fixe (103) et comportant au moins un élément souple (106) qui empêche ladite première couronne de comptage (110) de tourner en sens inverse du sens de rotation imposé par ledit organe d'entraînement (130).

21. Dispositif de comptage selon l'une quelconque des revendications 12 à 20, dans lequel ladite languette d'entraînement (114) s'étend circonférentiellement par rapport audit axe de rotation fixe (103), sa surface intérieur formant environ une surface annulaire (118), et comporte à une extrémité une tête (115), mobile radialement entre une position de repos, où ladite tête (115) s'étend radialement vers l'intérieur au-delà de ladite surface annulaire intérieure (118), et une position d'entraînement, où ladite tête (115) coopère avec ladite seconde couronne de comptage (120), ledit moyen de came (108) étant solidaire dudit élément tubulaire fixe (104) et disposé sans frottement sensiblement contre ladite surface annulaire intérieure (118) de ladite languette d'entraînement (114), pour forcer la tête (115) de ladite languette (114) dans sa position d'entraînement, chaque fois que ladite première couronne de comptage (110) effectue un tour complet autour dudit axe de rotation (103).

22. Dispositif de comptage selon la revendication 21, dans lequel ladite seconde couronne de comptage (120) comporte une série de dents (121) s'étendant circonférentiellement par rapport audit axe de rotation fixe (103) et tournées vers l'intérieur, ladite série de dents (121) étant disposée radialement à l'extérieur de ladite languette d'entraînement (114) de ladite première couronne de comptage (110), de sorte que, dans sa position d'entraînement, ladite tête (115) de la languette (114) s'enclenche dans une dent de ladite série de dents (121) pour entraîner ladite seconde couronne de comptage (120) en rotation autour dudit axe de rotation fixe (103).

23. Dispositif de comptage selon l'une quelconque des revendications précédentes, dans lequel les premières et secondes couronnes de comptage (10, 110) et (20, 120) comportent des moyens d'affichage sur leurs surfaces périphériques extérieures respectives (16, 116) et (26, 126).

## Patentansprüche

1. Vorrichtung zum Zählen der Dosissen eines Produktes, die von einer Abgabevorrichtung für flüssige oder pulverförmige Produkte abgegeben werden, wobei die Zählvorrichtung einen ersten Zählkranz (10, 110) und einen zweiten Zählkranz (20, 120) umfaßt und die beiden Zählkränze in drehbarer Weise um eine feste Drehachse (3, 103) montiert sind, wobei der erste Zählkranz (10, 110) eine bezüglich der festen Rotationsachse (3, 103) in Umfangsrichtung angeordnete Zahnung (11, 111) umfaßt, die mit einem Antriebsorgan (30, 130) zusammenwirkt, das dazu dient, den ersten Zählkranz um die feste Rotationsachse bei jeder Betätigung der Abgabevorrichtung zu drehen, wobei der erste Zählkranz (10, 110) weiterhin eine Antriebszunge (14, 114) umfaßt, die zwischen einer Ruhelage, in welcher sie nicht mit dem zweiten Zählkranz (20, 120) zusammenwirkt, und einer Antriebslage bewegbar ist, in welcher sie mit dem zweiten Zählkranz (20, 120) zusammenwirkt, um diesen um die feste Rotationsachse zu drehen, wobei die Antriebszunge (14, 114) in ihre Antriebsposition vermittels eines Nocken (8, 108) gedrückt wird, dadurch gekennzeichnet, daß der zweite Zählkranz (20, 120) nach der Abgabe einer vorbestimmten Anzahl von Dosissen mit einer festen Anschlagsvorrichtung (52, 152) zusammenarbeitet, die jegliche weitere Drehung des zweiten Zählkranzes (20, 120) verhindert.

2. Zählvorrichtung nach Anspruch 1, bei der der zweite Zählkranz (20, 120) eine Reihe von Zacken (21, 121) umfaßt, die in Umfangsrichtung bezüglich der festen Drehachse (3, 103) angeordnet sind, und wobei die Antriebszunge (14, 114) des ersten Zählkranzes (10, 110) an einem Ende einen Kopf (15, 115) umfaßt, der in die Reihe von Zähnen (21, 121) des zweiten Zählkranzes einklinken kann, wenn sich die Antriebszunge in ihrer Antriebsposition befindet.

3. Zählvorrichtung nach Anspruch 1 oder 2, bei der der erste Zählkranz (10, 110), der als Einheiten-Zähler arbeitet, eine periphere Zahnung (11, 111) besitzt, die zehn Zähne aufweist, wobei diese zehn Zähne gleichmäßig um die feste Achse (3, 103) herum verteilt angeordnet sind, wobei die Antriebszunge (14, 114) mit der Nockeneinrichtung (8, 108) für einen Rotationsantrieb des zweiten Zählkranzes (20, 120), der als Zehner-Zähler arbeitet, immer dann zusammenwirkt, wenn der erste Zählkranz eine vollständige Umdrehung um die feste Rotationsachse ausführt.

4. Zählvorrichtung nach einem der vorhergehenden Ansprüche, bei der eine erste Blockiervorrichtung (6, 106), die auf den ersten Zählkranz (10, 110) einwirkt, um ihn daran zu hindern, sich in der Richtung zu drehen, die der Drehrichtung entgegengesetzt ist, die durch das Antriebselement (30, 130) erzeugt wird, und eine zweite Blockiereinrichtung (23, 51; 123, 151) umfaßt, die auf den zweiten Zählkranz (20, 120) einwirkt, um ihn daran zu hindern, sich in einer Richtung zu drehen, die der Drehrichtung entgegengesetzt ist, die durch die Antriebszunge (14, 114) des ersten Zählkranzes (10, 110) vorgegeben wird.

5. Zählvorrichtung nach Anspruch 4, bei der die zweite Blockiereinrichtung eine elastische Klaue (23, 123) umfaßt, die fest mit dem zweiten Zählkranz verbunden und an einem ihrer Enden mit einem Zapfen (22, 122) versehen ist, wobei dieser Zapfen mit einem geriffelten Profil (51, 151) zusammenwirkt, das bezüglich der Rotationsachse (103) feststehend ist, um den zweiten Zählkranz daran zu hindern, sich in irgendeiner Richtung zu drehen, wenn sich die Antriebszunge (14, 114) des ersten Zählkranzes in ihrer Ruheposition befindet.

6. Zählvorrichtung nach Anspruch 5, bei der das feste geriffelte Profil (51, 151), das mit dem Zapfen (22, 122) der elastischen Klaue (23, 123) der zweiten Blockiervorrichtung zusammenwirkt, die feste Anschlagseinrichtung (52, 152) umfaßt, die den Zapfen (22, 122) der elastischen Klaue blockiert und so eine Drehung des zweiten Zählkranzes verhindert, wobei die maximale Anzahl der Dosissen, die von der Abgabevorrichtung emitiert werden, auf diese Weise durch die Anzahl der Rillen des geriffelten Profils festgelegt wird, die sich vor der Anschlagsvorrichtung (52, 152) befinden.

7. Zählvorrichtung nach einem der vorhergehenden Ansprüche, bei der eine Stange (4) fest auf der festen Rotationsachse (3) montiert ist und die im wesentlichen ringförmigen ersten und zweiten Zählkränze (10, 20) in drehbarer Weise auf der festen Stange (4) montiert sind, wobei der erste Zählkranz (10) eine periphere Zahnung (11) umfaßt, die sich bezüglich der festen Achse (3) in Umfangsrichtung erstreckt und deren Zähne nach außen gerichtet sind, wobei die Zahnung (11) mit einem Antriebsorgan (30) zusammenwirkt, das fest mit einem Betätigungsstössel der Abgabevorrichtung verbunden ist und eine Translationsbewegung ausübt, wobei das Antriebselement (30) bei jeder Betätigung des Stössels mit einem Zahn der Zahnung (11) zusammenwirkt, um den ersten Zählkranz (10) um die feste Rotationsachse (3) zu drehen.

8. Zählvorrichtung nach Anspruch 7, bei der eine erste Blockiervorrichtung vorgesehen ist, die eine feststehende elastische Zunge (6) umfaßt, die mit der Zahnung (11) des ersten Zählringes (10) zusammenarbeitet, um diesen daran zu hindern, sich in einer Richtung zu drehen, die der Drehrichtung entgegengesetzt ist, die durch das Antriebsorgan (30) vorgegeben wird.

9. Zählvorrichtung nach Anspruch 7 oder 8, bei der der erste Zählkranz (10) eine Antriebszunge (14) umfaßt, die sich bezüglich der feststehenden Rotationsachse (3) in Umfangsrichtung erstreckt und an einem Ende einen Kopf (15) besitzt, der radial zwischen einer Ruhelage, in der der Kopf (15) sich radial nach außen über die ringförmige Außen-Oberfläche (18) des ersten Zählkranzes (10) hinaus erstreckt, und einer Antriebsposition beweglich ist, in welcher der Kopf (15) mit dem zweiten Zählkranz (20) zusammenwirkt, wobei die Nockeneinrichtung (8) bezüglich der Rotationsachse (3) fest und ohne wesentliche Reibung an der äußeren ringförmigen Oberfläche (18) des ersten Zählkranzes (10) in Höhe der Antriebszunge (14) angeordnet ist, um den Kopf (15) der Antriebszunge (14) in seine Antriebsposition jedesmal dann zu drücken, wenn der erste Zählkranz (10) eine vollständige Drehung um die feste Rotationsachse (3) ausführt.

10. Zählvorrichtung nach Anspruch 9, bei der der zweite Zählkranz (20) eine Reihe von Zähnen umfaßt, die sich bezüglich der festen Rotationsachse (3) in Umfangsrichtung erstreckt und diese Zähne nach außen gerichtet sind, wobei die Reihe von Zähnen radial innerhalb der Antriebszunge (14) des ersten Zählkranzes in der Weise angeordnet ist, daß der Kopf (15) der Zunge (14) in seiner Antriebsposition in einen Zahn der besagten Zahnreihe eingreift, um den zweiten Zählkranz (20) für eine Drehung um die feste Rotationsachse (3) anzutreiben.

11. Zählvorrichtung nach einem der vorhergehenden Ansprüche, bei der die Außenseite des Kopfes (15) der Antriebszunge (14) ein zum Profil der feststehenden Nockeneinrichtung (8) komplementäres Profil besitzt und die Innenseite des Kopfes (15) ein Profil besitzt, das zum Profil der Zähne der besagten Zahnreihe des zweiten Zählkranzes (20) komplementär ist.

12. Zählvorrichtung nach einem der Ansprüche 1 bis 6, bei der der erste Zählkranz (110) eine erste Zahnung (111) umfaßt, die sich bezüglich der feststehenden Rotationsachse (103) in Umfangsrichtung erstreckt und deren Zähne, die nach außen gerichtet sind, um die besagte feste Achse (103) regelmäßig herum verteilt angeordnet sind, um mit einem Antriebsorgan (130) zusammenwirken, das eine Drehbewegung ausübt, wobei das Antriebsorgan (130) von einem Verwender vermittels eines Betätigungsknopfes betätigt wird, der um die Rotationsachse (103) zwischen einer ersten und einer zweiten Extremstellung drehbar ist.

13. Zählvorrichtung nach Anspruch 12, bei der das Antriebsorgan (130) ringförmig und um die besagte feststehende Rotationsachse (103) drehbar montiert ist sowie einen ringförmigen elastischen Arm (131) umfaßt, der in Radialrichtung zwischen einer Ruhelage, in der ein vorspringender Teil (132) des Arms (131) sich in Radialrichtung nach außen über die äußere ringförmige Oberfläche des Armes (131) hinaus erstreckt, und einer Antriebsposition beweglich ist, in der der vorspringende Teil (132) des Arms (131) mit einem Zahn der ersten Zahnung (111) des ersten Zählkranzes (110) zusammenwirkt, um diesen für eine Drehung anzutreiben, wobei der elastische Arm (131) vom Betätigungsknopf (140) in seine Antriebsposition gedrückt wird.

14. Zählvorrichtung nach Anspruch 13, bei der der Betätigungsknopf (140) ringförmig und in drehbarer Weise um die besagte feststehende Rotationsachse (103) in der Weise montiert ist, daß er das Antriebsorgan (130) umgibt, wobei der Betätigungsknopf (140) Einrichtungen (142) umfaßt, die dazu dienen, das Antriebsorgan (130) um die besagte feststehende Rotationsachse (103) zu drehen, sowie Einrichtungen (141), um den Arm (131) in seine Antriebsposition zu drücken.

15. Zählvorrichtung nach Anspruch 14, bei der die Einrichtungen, die dazu dienen, den Arm (131) in seine Antriebsposition zu drücken, eine Ausbauchung (141) umfassen, die an der inneren ringförmigen Fläche des Betätigungsknopfes (140) angeordnet ist, und wobei die Einrichtungen, die dazu dienen, das Antriebsorgan (131) zu drehen, zwei Vorsprünge (142a, 142b) umfassen, die mit dem Antriebsorgan (130) zusammenwirken, wobei die beiden Vorsprünge (142a, 142b) in gleicher Höhe über die innere ringförmige Oberfläche des Betätigungsknopfes (140) vorspringen, wobei der erste Vorsprung dazu geeignet ist, das Antriebsorgan (130) in einer Richtung zu drehen, um den vorspringenden Teil (132) seines Arms (131) in eine Position gegenüber einem Zahn der ersten Zahnung (111) mitzunehmen, und der zweite Vorsprung (142b) geeignet ist, das Antriebsorgan (130) in der anderen Richtung anzutreiben, um den ersten Zählkranz (110) zu drehen, wenn sich der elastische Arm (131) in seiner Antriebsposition befindet.

16. Zählvorrichtung nach Anspruch 15, bei der die erste Zahnung (111) des ersten Zählkranzes (110) zehn Zähne umfaßt, wobei der Winkelabstand zwischen den zwei Extrempositionen des Betätigungsknopfes (140) ungefähr 180° beträgt, und die beiden Vorsprünge (142a, 142b) mit einem Winkelabstand von ungefähr 144° voneinander angeordnet sind, wobei der Betätigungsknopf (140) zunächst um 180° in einer Richtung zu seiner zweiten Extremposition hin gedreht wird, um den vorspringenden Teil (132) des Arms (131) des Antrieborganes (130) in eine Position gegenüber dem folgenden Zahn der ersten Zahnung (111) mitzunehmen, und dann in seine zweite Extremposition zurückgebracht wird, in dem er in der entgegengesetzten Richtung gedreht wird, wodurch der zweite Vorspung (142b) das Antriebsorgan (130) antreibt, und wobei der Arm (131) in seine Antriebsposition gedrückt wird, um den ersten Zählkranz (110) um die Rotationsachse (103) zu drehen.

17. Zählvorrichtung nach Anspruch 16, bei der die Zählvorrichtung weiterhin eine als "Gesamthubvorrichtung" bezeichnete Vorrichtung umfaßt, die verhindert, daß der Betätigungsknopf (140) in seine Anfangsposition zurückgebracht wird, wenn er nicht zuvor bis zu seiner Anschlagsvorrichtung gedreht worden ist, um eine korrekte Positionierung des vorspringenden Teils (132) des Arms (131) gegenüber einem Zahn der ersten Zahnung (111) sicherzustellen.

18. Zählvorrichtung nach Anspruch 17 bei der die Gesamthubvorrichtung eine feste Platte (160) umfaßt, die fest mit der feststehenden Rotationsachse (103) verbunden ist und eine im wesentlichen ringförmige Schiene (161) umfaßt, die sich bezüglich der Rotationsachse (103) in Umfangsrichtung über ungefähr 180° erstreckt, sowie eine Klinke (165), die mit einem elastischen Finger (166) versehen ist, wobei die Klinke (165) drehfest mit dem Betätigungsknopf 140) verbunden ist und der elastische Finger (166) in der Anfangsposition des Betätigungsknopfes in das Innere der Schiene gezwungen wird, wobei die Schiene (161) eine Treppenwange (164) umfaßt, die mit dem elastischen Finger (166) zusammenwirkt, um eine Drehung in entgegengesetzter Richtung des Betätigungsknopfes (140) zu verhindern, wobei der elastische Finger (166) aus der Schiene (161) an ihrem einen Ende (163) austritt, um das Zurückkehren des Betätigungsknopfes (140) in seine Anfangsposition zu ermöglichen.

19. Zählvorrichtung nach Anspruch 18, bei der das Ende (163) der Schiene (161) eine Anschlagseinrichtung bildet, die die zweite Extremposition des Betätigungsknopfes definiert.

20. Zählvorrichtung nach einem der vorhergehenden Ansprüche 12 bis 19, bei der der erste Zählkranz (110) eine zweite Zahnung (107) umfaßt, die sich bezüglich der festen Rotationsachse (103) in Umfangsrichtung erstreckt und deren Zähne, die nach innen gerichtet sind, in regelmäßigen Abständen um die feste Rotationsachse (103) herum angeordnet sind, um mit einer ersten Blockiervorrichtung zusammenzuwirken, die fest mit einem festen rohrförmigen Element (104) verbunden ist, das auf der festen Rotationsachse (103) montiert ist und wenigstens ein elastisches Element (106) umfaßt, das verhindert, daß sich der erste Zählkranz (110) in einer Richtung dreht, die zu der durch das Antriebselement (130) vorgegebenen Drehrichtung entgegengesetzt ist.

21. Zählvorrichtung nach einem der Ansprüche 12 bis 20, bei der sich die Antriebszunge (114) in Umfangsrichtung bezüglich der festen Rotationsachse (103) erstreckt, wobei ihre Innenfläche ungefähr eine ringförmige Oberfläche (118) bildet, und sie an einem Ende einen Kopf (115) umfaßt, der in radialer Richtung zwischen einer Ruhelage, in der sich der Kopf (115) radial nach innen über die besagte innere ringförmige Oberfläche (118) hinaus erstreckt, und einer Antriebsposition beweglich ist, in der der Kopf (115) mit dem zweiten Zählkranz (120) zusammenwirkt, wobei die Nockeneinrichtung (108) fest mit dem festen rohrförmigen Element (104) verbunden und ohne wesentliche Reibung an der inneren ringförmigen Oberfläche (118) der Antriebszunge (114) anliegt, um den Kopf (115) der Zunge (114) jedesmal dann in seine Antriebsposition zu drücken, wenn der erste Zählkranz (110) eine vollständige Umdrehung um die besagte Rotationsachse (103) ausführt.

22. Zählvorrichtung nach Anspruch 21, bei der der zweite Zählkranz (120) eine Reihe von Zähnen (121) umfaßt, die sich bezüglich der festen Rotationsachse (103) in Umfangsrichtung erstrecken und nach innen gerichtet sind, wobei diese Reihe von Zähnen (121) radial außerhalb der Antriebszunge (114) des ersten Zählkranzes (110) derart angeordnet ist, daß der Kopf (115) der Zunge (114) in seiner Antriebsposition in einen Zahn der besagten Zahnreihe (121) eingreift, um den zweiten Zählkranz (120) um die besagte feststehende Achse (103) zu drehen.

23. Zählvorrichtung nach einem der vorhergehenden Ansprüche, wobei die ersten und zweiten Zählkränze (10, 110 und 20,120) Anschlagmittel an ihren jeweiligen peripheren äußeren Oberflächen (16,116 und 26,126) umfassen.

## Claims

1. A device for counting doses of substance issued by a dispenser for fluids or powders, the device comprising a first count wheel (10, 110) and a second count wheel (20, 120), both count wheels being mounted to rotate about a fixed axis of rotation (3, 103), said first count wheel (10, 110) comprising a set of teeth (11, 111) extending circumferentially about said fixed axis of rotation (3, 103) and co-operating with a drive member (30, 130) for rotating said first count wheel about said fixed axis of rotation on each use of the dispenser, said first count wheel (10, 110) further including a drive tongue (14, 114) that is movable between a rest position in which it does not co-operate with said second count wheel (20, 120), and a drive position in which it does co-operate with said second count wheel (20, 120) to cause it to rotate about said fixed axis of rotation, said drive tongue (14, 114) being forced into its drive position by cam means (8, 108), the device being characterized in that said second count wheel (20, 120) co-operates, after issuing a predetermined number of doses, with fixed abutment means (52, 152) which prevent any subsequent rotation of said second count wheel (20, 120).

2. A counter device according to claim 1, in which said second count wheel (20, 120) includes a set of teeth (21, 121) disposed circumferentially about said fixed axis of rotation (3, 103) and said drive tongue (14, 114) of said first count wheel (10, 110) includes a head (15, 115) at one end, said head engaging in said set of teeth (21, 121) of said second count wheel when said drive tongue is in its drive position.

3. A counter device according to claim 1 or claim 2, in which said first count wheel (10, 110) acts as a units counter and includes a peripheral set of teeth (11, 111) containing ten teeth, said ten teeth being uniformly distributed around said fixed axis (3, 103), and each time said first count wheel has performed a complete revolution about said fixed axis of rotation said drive tongue (14, 114) co-operates with said cam means (8, 108) to rotate said second count wheel (20, 120) which acts as a tens counter.

4. A counter device according to any preceding claim, in which there are provided a first locking device (6, 106) acting on the first count wheel (10, 110) to prevent it from returning in the direction opposite to the direction of rotation imposed by said drive element (30, 130), and a second locking device (23, 51; 123, 151) acting on said second count wheel (20, 120) to prevent it from rotating in the direction opposite to the direction of rotation imposed by said drive tongue (14, 114) of said first count wheel (10, 110).

5. A counter device according to claim 4, in which said second locking device comprises a flexible leg (23, 123) secured to said second count wheel and provided at its one of its ends with a stud (22, 122), said stud co-operating with a grooved profile (51, 151) that is fixed relative to the axis of rotation (103) to prevent said second count wheel from rotating in either direction when said drive tongue (14, 114) of said first count wheel is in its rest position.

6. A counter device according to claim 5, in which said fixed grooved profile (51, 151) which co-operates with said stud (22, 122) of said flexible leg (23, 123) of said second locking device includes said fixed abutment means (52, 152) locking said stud (22, 122) of said flexible leg, thus preventing rotation of said second count wheel, the maximum number of doses issued by the dispenser thus being determined by the number of grooves in the grooved profile situated ahead of said abutment means (52, 152).

7. A counter device according to any preceding claim, in which a rod (4) is mounted in fixed manner on said fixed axis of rotation (3) and the first and second count wheels (10, 20) are substantially annular and are mounted to rotate on said fixed rod (4), said first count wheel (10) having a peripheral set of teeth (11) extending circumferentially about said fixed axis (3) with the teeth thereof being outwardly directed, said set of teeth (11) co-operating with a drive member (30) secured to an actuator pushbutton of the dispenser and exerting movement in translation, said drive element (30) co-operating on each actuation of the pushbutton with a tooth of said set of teeth (11) to cause said first count wheel (10) to rotate about said fixed axis or rotation (3).

8. A counter device according to claim 7, in which there is provided a first locking device including a fixed flexible blade (6) which co-operates with the set of teeth (11) of said first count wheel (10) to prevent it from rotating in the direction opposite to the direction of rotation imposed by said drive member (30).

9. A counter device according to claim 7 or claim 8, in which said first count wheel (10) includes a drive tongue (14) which extends circumferentially about said fixed axis of rotation (3) and which includes a head (15) at one end, the head being radially movable between a rest position in which said head (15) extends radially inwards beyond the outer annular surface (18) of the first count wheel (10), and a drive position in which the head (15) co-operates with said second count wheel (20), said cam means (8) being fixed relative to said axis of rotation (3) and being disposed without friction substantially against said outer annular surface (18) of said first count wheel (10) level with said drive tongue (14) to force said head (15) of said drive tongue (14) into its drive position each time said first count wheel (10) has performed one complete revolution about said fixed axis of rotation (3).

10. A counter device according to claim 9, in which said second count wheel (20) includes a set of teeth extending circumferentially about said fixed axis of rotation (3) and facing outwardly, said set of teeth being disposed radially inside said drive tongue (14) of said first count wheel in such a manner that when said head (5) of the tongue (14) is in its drive position it engages in a tooth of said set of teeth to drive said second count wheel (20) to rotate about said fixed axis of rotation (3).

11. A counter device according to claim 10, in which the outside of said head (15) of the drive tongue (14) has a profile complementary to the profile of said fixed cam means (8), and the inside of said head (15) has a profile that is complementary to the profile of said teeth of said set of teeth of the second count wheel (20).

12. A counter device according to any one of claims 1 to 6, in which said first count wheel (110) includes a first set of teeth (111) extending circumferentially about said fixed axis of rotation (103) and having its teeth inwardly directed and uniformly distributed around said fixed axis (103) to co-operate with a drive member (130) exerting rotary movement, said drive member (130) being actuated by the user by means of an actuator knob that is movable in rotation about the axis of rotation (103) between first and second extreme positions.

13. A counter device according to claim 12, in which said drive member (130) is annular, and mounted to rotate about said fixed axis of rotation (103), and includes an annular flexible arm (131) movable radially between a rest position in which a projecting portion (132) of said arm (131) extends radially outwards beyond the annular outer surface of said arm (131), and a drive position in which said projecting portion (132) of the arm (131) co-operates with a tooth of said first set of teeth (111) of said first count wheel (110) to drive it in rotation, said flexible arm (131) being forced into its drive position by the actuator knob (140).

14. A counter device according to claim 13, in which said actuator knob (140) is annular and is mounted to rotate about said fixed axis of rotation (103) in such a manner as to surround said drive member (130), said actuator knob (140) including means (142) for rotating said drive member (130) about said fixed axis of rotation (103), and means (141) for forcing said arm (131) into its drive position.

15. A counter device according to claim 14, in which said means for forcing said arm (131) into its drive position include a swelling (141) disposed on the inner annular face of said actuator knob (140), and said means for rotating the drive member (131) include two projections (142a, 142b) which co-operate with said drive member (130), both projections (142a, 142b) being disposed at the same height on the inner annular face of said actuator knob (140), the first projection being adapted to cause the drive member (131) to rotate in one direction to bring the projecting portion (132) of its arm (131) face to face with a tooth of said first set of teeth (111), and the second projection (142b) being adapted to drive the drive member (130) in the other direction to rotate said first count wheel (110) when the flexible arm (131) is in its drive position.

16. A counter device according to claim 15, in which said first set of teeth of said first count wheel (110) has ten teeth, the angular distance between the two extreme positions of the actuator knob (140) being about 180°, and said projections (142a, 142b) being disposed so as to be angularly spaced apart by about 144°, said actuator knob (140) being initially rotated through 180° in one direction towards its second extreme position to bring said projecting portion (132) of the arm (131) of the drive member (130) face to face with the next tooth in the first set of teeth (111) and then being returned to its first extreme position by being rotated in the opposite direction, the second projection (142b) rotating said drive member (130), and in which said arm (131) is forced into its drive position to cause said first count wheel (110) to rotate about the axis of rotation (103).

17. A counter device according to claim 16, in which said counter device further includes a full stroke device preventing said actuator knob (140) from being returned to its initial position unless it has previously been rotated as far as its stop means, so as to ensure that said projecting portion (132) of said arm (131) is properly positioned facing a tooth of said first set of teeth (111).

18. A counter device according to claim 17, in which said full stroke device comprises a fixed plate (160) secured to said fixed axis of rotation (103) and supporting a substantially annular rail (161) extending circumferentially about said axis of rotation (103) through about 180°, and a pawl (165) provided with a flexible finger (166), said pawl (165) being constrained to rotate with said actuator knob (140), said flexible finger (166) being constrained in the initial position of the actuator knob to pass inside said rail, said rail (161) including a rack (164) co-operating with said flexible finger (166) to prevent said actuator knob (140) rotating in an opposite direction, said flexible finger (166) exiting said rail (161) at an end (163) thereof to enable said actuator knob (140) to return to its initial position.

19. A counter device according to claim 18, in which said end (163) of said rail (161) forms stop means defining the second extreme position of said actuator knob.

20. A counter device according to any one of claims 12 to 19, in which said first count wheel (110) includes a second set of teeth (107) extending circumferentially about said fixed axis of rotation (103) and having its inwardly extending teeth uniformly distributed about said fixed axis of rotation (103) to co-operate with a first locking device secured to a fixed tubular element (104) fixedly mounted on said fixed axis of rotation (103) and including at least one flexible element (106) preventing said first count wheel (110) from rotating in the direction opposite to the direction of rotation imposed by said drive member (130).

21. A counter device according to any one of claims 12 to 20, in which said drive tongue (114) extends circumferentially about said fixed axis of rotation (103), its inner surface approximately forming an annular surface (118), and includes at one end a head (115) that is radially movable between a rest position in which the head (115) extends radially inwards beyond said inner annular surface (118) and a drive position in which said head (105) co-operates with said second count wheel (120), said cam means (108) being secured to said fixed tubular element (104) and disposed without significant friction against said inner annular surface (118) of said drive tongue (114) to force the head (115) of said tongue (114) into its drive position whenever said first count wheel (110) has performed a complete revolution about said axis of rotation (103).

22. A counter device according to claim 21, in which said second count wheel (120) includes a set of teeth (121) extending circumferentially about said fixed axis of rotation (103) and directed inwardly, said set of teeth (121) being disposed radially outside said drive tongue (114) of said first drive wheel (110) in such a manner that in its drive position said head (115) of the tongue (114) engages in one of the teeth of said set of teeth (121) to drive said second count wheel (120) in rotation about said fixed axis of rotation (103).

23. A counter device according to any preceding claim, in which the first and second count wheels (10, 110; 20, 120) include display means on their respective outer peripheral surfaces (16, 116; 26, 126).
